# EUROPEAN PATENT APPLICATION

(11) **EP 1 707 626 A1**
(43) Date of publication of application: **04.10.2006**
(21) Application number: 04820636.1
(22) Date of filing: 12.11.2004
(51) Int. Cl.: C12N 15/00, C12N 1/15, C12N 1/19, C12N 1/21, C12N 5/00, C12P 21/02, C12Q 1/68

(54) **GENE AMPLIFICATION METHOD**

(30) Priority: 25.11.2003 JP 2003394273
(71) Applicant: Japan Science and Technology Agency, Kawaguchi-shi, Saitama 332-0012 (JP)
(72) Inventor: HORIUCHI, Takashi, 4440841 (JP); WATANABE, Takaaki, 4440823 (JP)
(74) Representative: Wilson Gunn
(86) International application number: PCT/JP2004/016833
(87) International publication number: WO 2005/061703

(57) **Abstract**

The present invention provides a double-stranded DNA constructed specially for gene amplification at high speed, a method for gene amplification using the double-stranded DNA and a method to produce proteins.

A system to induce artificial gene amplification at high speed was constructed based on the mechanism of gene replication *in vivo* (BIR). A double-stranded DNA comprising an arrangement of A-B-C and A'-B'-C' or an inverted arrangement of A'-B'-C' was constructed. A and A' are double-stranded DNA capable of undergoing reciprocal homologous recombination and said DNA fragments are arranged each other in an inverted orientation; B and B' are amplifying segments, wherein at least one or the other of said genes contains a target gene for amplification; C and C' are double-stranded DNA capable of undergoing reciprocal homologous recombination, wherein said DNA fragments are arranged each other in an inverted orientation and any DNA sequence could be inserted between C and C'. B and B' may be eliminated and, in this case, A or C could be a target gene for amplification. The double-stranded DNA was integrated into a chromosome or a plasmid and induction of an enzyme to cut an arbitrarily specific sequence induces a break at a specific site, which triggered gene amplification at high speed.

## Description

### Technical field:

The present invention relates to a method for performing gene amplification at high speed and a method for preparing proteins using the amplified gene.

### Prior art:

Induction of artificial gene amplification in animal cell cultures (e.g. patent reference 1) accompanies some troubles such as (i) time-consuming (a half year to a year), (ii) accompanying many clones without amplification, (iii) dependence on empirical know-how without elucidation of amplification mechanism and others. On the other hand, a system using yeast for gene amplification is not available and usually a plasmid is used for the purpose with a limited copy numbers.

The system of the present invention based on a biological capability referred to as BIR (Break-Induced-Replication) (non-patent reference 1, 2). The biological capability is regarded as that of self-remedy of chromosome comprising finding the same chromosomal sequence when it is broken, invading the site using the homology, constructing a replication point and starting replication. All living organisms are regarded as having the capability.
Patent reference 1: WO94/14968.
Non-patent reference 1: PNAS, vol. 98, no.15, 8255-8262 (July 17, 2001).
Non-patent reference 2: Genes Dev 12, 3831-3842 (1998)

### Problems to be resolved by the invention:

The present invention provides a double-stranded DNA constructed specially for gene amplification at high speed and a method for gene amplification using the double-stranded DNA. The present invention has the characteristics in the complete construction of an artificial amplification system, in capability of synchronized amplification, in amplification during a short time (probably in a generation), and in elucidated mechanism of amplification.

### Means to solve the problems:

The present inventors constructed an artificial high-speed gene amplification system based on the mechanism of gene replication in living organism referred as BIR.

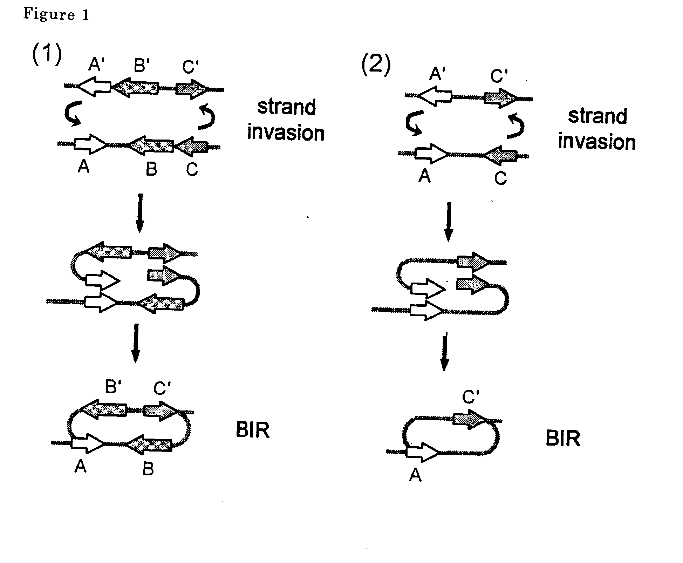

The present inventors constructed a first double-stranded DNA (A-B-C) containing a target gene (B) for amplification and gene segments (A and C) necessary for amplification located at both ends of the target gene (B), and a second double-stranded DNA constructed in the same way (A'-B'-C': B' may be the target gene for amplification, A and A', and C and C' are capable of undergoing reciprocal homologous recombination) as shown in Fig. 1 (1) and conducted a gene amplification test (herein, B and B' may be eliminated as in Fig. (2)). In this case, A or C could be a target gene for amplification.). As the results, it was confirmed that, when a break is induced at a specific site, this triggered a gene amplification in high speed (see Examples 2 to 4).

Namely, the present invention is a double-stranded DNA comprising (a) an arrangement of A-C and (b-1) an arrangement of A'-C' or (b-2) an inverted arrangement of A'-C', wherein A and A' are each double-stranded DNA and are capable of undergoing reciprocal homologous recombination and one of A and A' is an inverted orientation of the other, C and C' are each double-stranded DNA and are capable of undergoing reciprocal homologous recombination and one of C and C' is an inverted orientation of the other, and at least one of A and C comprises a target gene for amplification, and any DNA sequence may be inserted among A, A', C and C'.

Also, the present invention is a double-stranded DNA comprising (a) an arrangement of A-B-C and (b-1) an arrangement of A'-B'-C' or (b-2) an inverted arrangement of A'-B'-C', wherein A and A' are each double-stranded DNA and are capable of undergoing reciprocal homologous recombination and one of A and A' is an inverted orientation of the other, B and B' are amplifying segments where at least one of B and B' containing at least one target gene for amplification, C and C' are each double-stranded DNA and are capable of undergoing reciprocal homologous recombination and one of C and C' is an inverted orientation of the other, and any DNA sequence may be inserted among A, A', B, B', C and C'.

B and B' may be amplifying segments each containing at least one target gene for amplification arranged in the same orientation and are capable of undergoing reciprocal homologous recombination. Furthermore, B and B' may further contain a selection gene for amplification arranged in the same orientation.

The preferable examples of the double-stranded DNA include the following arrangements:
(1) A-C-A'-C',
(2) A-C-D-A'-C',
(3) (i) E'-A-C and (ii) A'-C'-E or an inverted arrangement of A'-C'-E, or (iii) A -C-E and (iv) E'-A'-C' or an inverted arrangement of E'-A'-C',
(4) D-E'-A-C-D-A'-C'-E-D, D-E'-A-C-D-E'-C"-A"-D, D-A-C-E-D-E'-A'-C'-D, D-A-C-E-D-C"-A"-E-D, or D-A-C-E-D-C"-A"-E-D,
(5) A-B-C-A'-B'-C',
(6) A-B-C-D-A'-B'-C',
(7) (i) E'-A-B-C and (ii) A'-B'-C'-E or an inverted arrangement of A'-B'-C'-E, or (iii) A-B-C-E and (iv) E'-A'-B'-C' or an inverted arrangement of E'-A'-B'-C', or
(8) D-E'-A-B-C-D-A'-B'-C'-E-D , D-E'-A-B-C-D-E'-C"-B"-A"-D , D-A-B-C-E-D-E'-A'-B'-C'-D, or D-A-B-C-E-D-C"-B "-A"-E-D

In these formula, D represents a double-stranded DNA fragment with at least one break point by endonuclease, E represents a telomere sequence, E' represents an inverted sequence of E and other symbols are the same as the above. C"-A" represents an inverted arrangement of A'-C', and C"-B"-A" represents an inverted arrangement of A'-B'-C'.

For example, (1) is practically identical to an arrangement of AC-C'A'-AC, which can be easily constructed by inversing the central arrangement to (C'A') after laying out three identical arrangements (AC). Furthermore, an insertion of a break site at the middle of the central arrangement (C'A') leads to AC-C'DA'-AC, which is identical to (2). Moreover, in these examples, A and C may be the same. For example, in this case, (1) becomes A-A-A'-A', (2) becomes A-A-D-A'-A', (5) becomes A-B-A-A'-B'-A', and (6) becomes A-B-A-D-A'-B'-A'.

Also, the present invention is a recombinant vector containing the double-stranded DNA of any one of above (1) to (8).

Furthermore, the present invention is a transformant transduced with the double-stranded DNA of the above (1), (2), (5) or (6). The double stranded-DNA of (1), (2), (5) or (6) is suitable to transduction into chromosomes.

Moreover, the present invention is a method for gene amplification comprising the steps of preparing the transformant and amplifying the target gene. (e.g. a target gene for amplification).

It is preferable that the transformant is treated with an endonuclease in the step of amplifying the target gene, when the double-stranded DNA is represented as A-C-D-A'-C' or A-B-C-D-A'-B'-C', wherein the symbols are the same as above.

Still furthermore, the present invention is a recombinant plasmid containing the double-stranded DNA of the above (3), (4), (7) or (8). The DNA of (3), (4), (7) or (8) is suitable for introducing into plasmid.

Still moreover, the present invention is a method for gene amplification comprising the steps of transducing bacteria with the plasmid of claim 15 and culturing the bacteria.

Also, the present invention is the method for producing a protein encoded by the target gene for amplification comprising the steps of culturing cells or bacteria obtained by any one of these methods.

### Brief description of the drawings:

Figure 1 shows the mechanism of BIR reaction.
Figure 2 shows a preferable construction of the double-stranded DNA of the present invention. A and A', and C and C' are sequences capable of undergoing reciprocal homologous recombination, B and B' are amplification segments, wherein at least one of them contains a target gene for amplification, D is a break site by endonuclease, and E and E' represent telomere sequences. The direction of the array represents the orientation of the arrangement of genes. In case of elimination ofB and B', A or C may be a target gene for amplification.
Figure 3 shows a process for gene amplification of the present invention. CEN represents centromere and TEL represents telomere. DSB (Double Strand Break) represents the site of double strand break.
Figure 4 shows a termination of the gene amplification.
Figure 5 shows the result of structural analysis of amplified product by Southern blotting. (A) shows the result after PFGE separation and (B) shows that after XhoI digestion. The number of the panel (A) shows the number of clones.
Figure 6 shows the result of structural analysis of amplified product by Southern blotting. (A) shows the result detected by the use of RET2 probe after separation by PFGE and (B) shows similarly that detected by the use of leu2d probe.
Figure 7 shows the number of the cells generated in Comparative Example 1 and Example 2. The abscissa shows the number of colonies generated by HO induction per 10⁵ viable cells.
Figure 8 shows the photographs of colonies generated in Comparative Example 1 and Example 2.
Figure 9 shows an amplification unit used in Comparative Example 1.
Figure 10 shows the construction of the double-stranded DNA used in Example 3.
Figure 11 shows the result of structural analysis of amplified product by Southern blotting. (A) shows the result for PFGE separation and (B) shows that after XhoI digestion. The number on the panel (A) represents the number of clones.
Figure 12 shows the organization of plasmid pBIA and pNotBIA used for Example 4.
Figure 13 shows the result of structural analysis of amplified product by Southern blotting. (A) shows the result for PFGE separation and (B) shows that after XhoI digestion. The number on the panel (A) represents the number of clones.
Figure 14 shows the amplified product for plasmid pBIA.
Figure 15 shows the number of Leu⁺Ura⁺ colonies obtained from cell strains (LS20) containing plasmids (pBIA and pNotBIA).

### Detailed description of the invention:

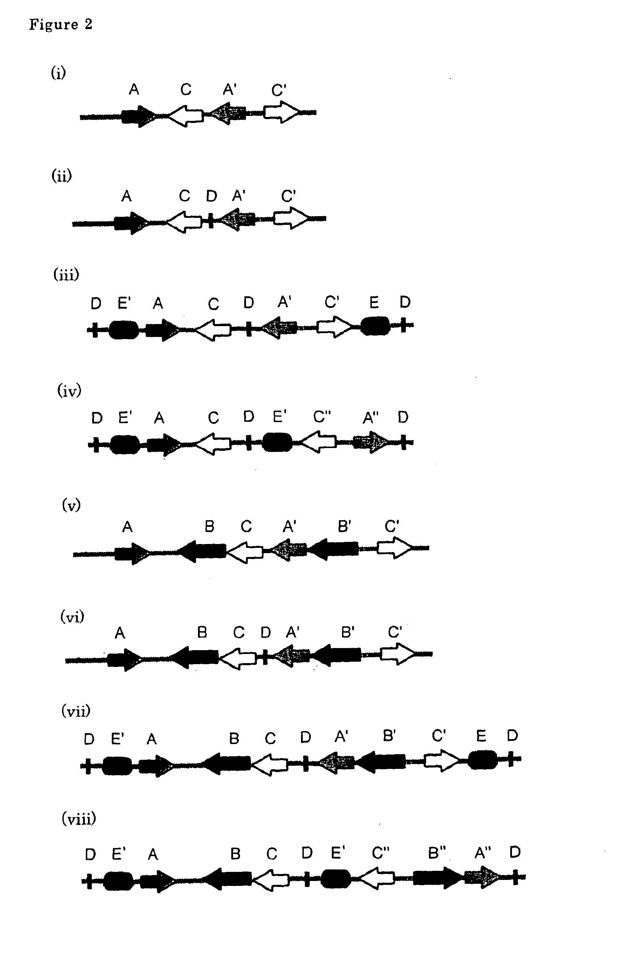

The double-stranded DNA of the present invention for gene amplification is represented by the arrangement of
(i) A-B-C and
(ii) A'-B'-C' or an inverted arrangement of A'-B'-C' (in the formula, B and B' may be eliminated. In this case, A or C may be a target gene for amplification.), and preferably any double-stranded DNA of the above (1) to (8).
   Some of the examples are shown in Fig. 2.

In the present description, these double-stranded DNA comprises the nucleotide sequence presented from 5'-end to 3'-end (from left to right). The combination of each constituent can be performed by a conventional art of genetic engineering.

A and A' are each double-stranded DNA, capable of undergoing reciprocal homologous recombination and preferably with the identical nucleotide sequence. Preferably, the sequences of A and A' are more than 90% homologous. Furthermore, particularly a proximal part (e.g. 10 to 30 bp) to a break site in A and A' (e.g. between C-A' or D) has preferably highly homologous. Moreover, the length of A and A' is preferably more than 500 bp. Short fragments could produce many by-products after gene amplification and could lead to homologous recombination at an unwilled site.

Still furthermore, A and A' are arranged each other in an inverted orientation. The relation of C and C' have the similar to that of A and A'. Also, A may be identical to C.

At least one of B and B', and preferably both B and B', are amplification segments and each of them may contain only a kind of target gene for amplification, but also may contain multiple target genes for amplification. At least a kind of target gene for amplification is preferably capable of undergoing reciprocal homologous recombination and more preferably identical gene.

Any gene could be used as said target gene for amplification. Principally, there is no limit in the gene length for stable amplification and amplification is possible for gene with more than 10 kb.

Furthermore, said target gene for amplification preferably contains at least one selection gene for amplification, and moreover, B and B' more preferably contain selection genes for amplification arranged in the same orientation. The selection gene for amplification includes leu2d, and promoter-deleted trp1, ura3, DFR1 gene and others as described later.

The B and B' may be eliminated. In this case, at least one of A and C may be the target gene for amplification.

D is a break recognition sequence of HO endonuclease (hereinafter, referred to as "HOcs") and has at least one break site. It can be replaced with other endonuclease (I-SceI and others) without recognition sequence in the genome (or with a few deletable recognition sequence) in combination with the break recognition sequence. By the replacement, the amplification efficiency could be markedly increased. D may contains two break sites and a marker gene between them.

HO endonuclease and I-SceI enzyme, which recognize longer sequence than usual endonuclease recognizing sequence of about 5 bp, are preferably used as an endonuclease.

E is a telomere sequence (hereinafter, it is referred to as "TEL"). Telomere sequence is a simple reiterated DNA sequence capable of forming telomere and need not to be identical sequence in spite of representing by the same symbol E. A telomere enables an amplified product to be maintained as a linear chromosome in a yeast cell. E' is an inverted sequence of E.

Also, the distance between A and C is preferably approximately the same as that between A' and C' in double-stranded DNA of the present invention based on the efficiency of the amplification. Therefore, and because of other reasons, any DNA sequence may be inserted between the above elements. Examples of such inserts include centromere (hereinafter, referred to as "CEN"), autonomously replicating sequence (hereinafter, referred to as "ARS"), selection markers and others.

CEN sequence may be any centromere sequence on a chromosome (in a following example, it is no. 4 chromosome). The selection pressure becomes stronger by CEN and amplified products could be kept stably. One CEN sequence is preferably inserted in any site between A-B-C (or A-C) and TEL; or between A'-B'-C' (or A'-C') or its inverted arrangement and TEL. Since insertion of CEN and ARS excludes their containment in a vector, general conventional vectors (i.e. pBR322, pUC series, pBluescript series, pGEN series and others) are preferably used.

ARS sequence is a yeast replication origin and could be any ARS sequence of chromosomes. Amplification products are maintained by ARS sequence. One ARS sequence is preferably inserted in any site between A-B-C (or A-C) and TEL; or between A'-B'-C' (or A'-C') or its inverted arrangement and TEL.
Selection markers include URA3, HIS3, TRP1, ADE2, LYS2 and others. The selection marker is preferably a marker with a different selection method to the selection gene for amplification in B. The selection marker enables cells containing plasmid before start of amplification to be selected and enables amplification reaction to be performed efficiently.
The selection marker is preferably not inserted in a site between two kinds of structure element and HOcs, i.e. between A-B-C and TEL; or between A'-B'-C' or its inverted arrangement and TEL.

The double-stranded DNA thus constructed is transduced into bacteria, cells or individual animal, in which actual gene amplification is induced, before genes are amplified. The hosts include bacteria, such as E. coli and B. subtilis, yeast, insect cells, animal cells, plant cells, individual mammal and others, and yeast and animal cells are preferably used. These host cells are transformed with the double-stranded DNA of the present invention and with a vector including transcriptional initiation signal, such as promoter, enhancer and others, and furthermore, terminator sequence, if necessary, according to the conventional technique of genetic engineering.

Mono copy plasmid is preferably used as a plasmid carrying the double-stranded DNA for gene amplification. The mono copy plasmid is a plasmid containing CEN sequence and ARS sequence and involves a yeast mono copy vector (e.g. YCp50, pRS313, pRS314,pRS315, pRS316, pRS317, pRS412, pRS413, pRS414, pRS415,pRS416, pAUR112, pAUR123 and others) and others, as examples. Moreover, additional insertion of yeast replication origin ARS sequence and yeast CEN sequence could be performed using various conventional vectors (e.g. pBR322, pUC series (pUC18, pUC19), pBluescript series (pBluescript II), pGEM series (pGEM-3Zf) and others.)

Gene amplification could be carried out by transduction of the plasmid into bacteria and others. The bacteria include E. coli and eukaryotes include yeast and others.

Transduction of such double-stranded DNA as described in the sequences (1) and (5) into chromosome induces gene amplification as it is. In this case, randomly induced double-strand break may lead to an accidental break between C and A', which may bring about pairing between exposed homologous sequences (between A and A', C and C'), bring about a reaction called BIR at two sites at the same time, induce a rolling circle with two replication folks, and induce gene replication.

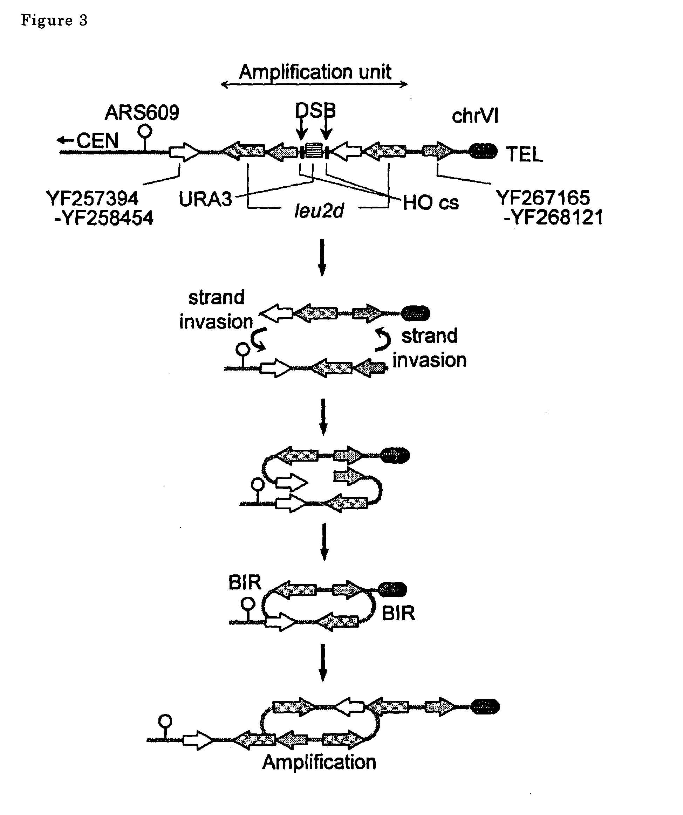

On the other hand, introduction of endonuclease is preferable for transduction of such sequences as described in the sequences (2) and (6) into chromosome. Introduction of endonuclease may induce break at a break site (D) in double-stranded DNA, which may bring about pairing between exposed homologous sequences (between A and A', C and C'), bring about a reaction called BIR at two sites at the same time, induce a rolling circle with two replication folks, and induce gene replication. Figure 3 interprets the process. Action of endonuclease could be performed by the introduction of this enzyme directly into cells or by the expression of a gene encoding the enzyme in cells. The time necessary for amplification is 12 to 24 hours.

Additionally, the frequency of emergence of amplified clones using those double-stranded DNA as described in the sequences (1) and (5) is lower than that using those as described in the sequences (2) and (6) together with endonuclease, and the former is about 1/5 of the latter. However, the time necessary for amplification is similar for both DNA.

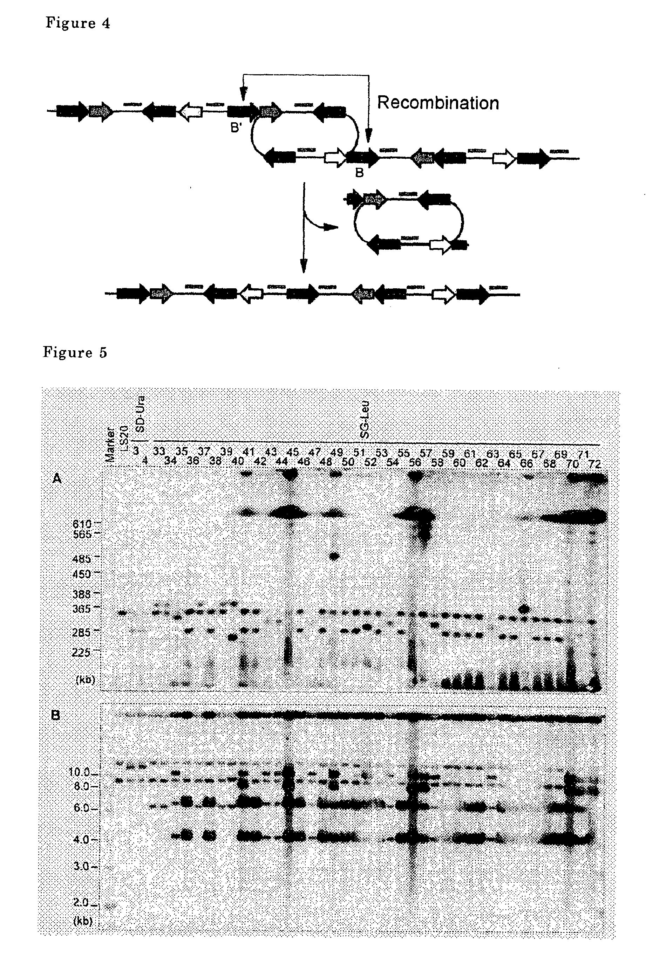

Essentially similar reaction as above will be took place for transduction of plasmid carrying the above sequences (3), (4), (7) and (8). The amplification time for plasmid carrying the sequence (4) and (8) is 1 to 24 hours.
In the final stage of the above reaction, after a recombination is induced between the amplified genes at two sites (B and B'), the above circle is removed and double-stranded DNA containing the amplified gene could be obtained (Fig. 4). Therefore, when B and B' are in opposite orientation or do not contain the sequence of the other, there will be no finalizing reaction removing the circle.

Also, a large amount of protein encoded the target gene for amplification will be produced after culturing the cells and purification from the culture media or supernatant.

### Industrial applicability of the invention:

By using double-stranded DNA of the present invention, gene amplification can be attained with highly efficiently and in a short time. Since the cells transduced with the double-stranded DNA of the present invention enables to produce a large amount of valuable proteins, the double-stranded DNA of the present invention is useful for preparing protein formulations and artificial antibodies. Furthermore, since these cells enable to keep gene expression in a large amount and in a stable state for long time, the double-stranded DNA of the present invention is also useful for gene therapy. Moreover, coupling gene amplification with mutagenesis enables to create a gene having an expected function.
The method of the present invention enables to prepare not only peptide and proteins without sugar side chains such as insulin but also highly productive micro-organisms producing erythropoietin, G-CSF, antibody agents and the like.

The following Examples illustrate the present invention, but are not intended to limit the scope of the invention.

### Example 1:

As shown the organization in the upper part of Fig. 3, an amplification unit, comprising URA3 gene (SEQ ID NO: 2), two kinds of budding yeast genome homologous sequence (YF257394-YF258454 (SEQ ID NO: 3) and YF267165-YF268121 (SEQ ID NO: 4), Gene bank Accession ID; NC001138), and selection marker gene for amplification lue2d (SEQ ID NO: 5) located between 2 HO sequences (DSB, SEQ ID NO: 1), was constructed. The selection marker gene for amplification leu2d plays a dual role as a target gene for amplification.

The selection marker gene leu2d for amplification is one of a group enzyme synthesizing leucine. Deletion of the gene inhibits cells to grow in a medium without leucine. Since the applied leu2d-deleted gene fails of most part of the promoter sequence, the expression level of the enzyme is very law, but a limited enzyme activity remained. Therefore, this one gene is not sufficient for cells growing in a medium without leucine, but amplification of the gene results in accumulation of the enzyme with weak activity and enables cells to grow in a medium without leucine. The feature was used for the selection of cells with amplified gene.

URA3 gene is used for selection of cells, wherein an amplification unit of a gene is kept without deletion in spite of a homologous recombination between leu2d genes.
A new replicating fork is generated after a double strand break of DNA is induced in HO sequence in an amplification unit and an end of exposed homologous sequence is paired with the homologous sequence of the genome. This BIR reaction takes place at two sites as shown in the middle of Fig. 3, and a replication of rolling cycle type may proceed. A segment inside the circle is amplified explosively in a cell cycle and the amplified product may be maintained inside the cells.

### Example 2:

The amplification unit constructed in example 1 was transduced into budding yeast cell strain (LS20), whose chromosome contains a structure with HO endonuclease gene inserted to a downstream of GAL promoter. An Ura⁺ colony, wherein an amplification unit of a gene is kept without deletion in spite of a homologous recombination between leu2d genes (marker genes), was isolated, and was spread on galactose media without leucine; and the expression of HO endonuclease was induced. Cells derived from emerged colony of Leu⁺ were cultured and chromosomal DNA was prepared in law-melting agarose gel. The culture time (including the time for growth of cells after amplification) was about 4 days.

The chromosomal DNA was separated by PFGE (pulsed-field gel electrophoresis) or by FIGE (field-inversion gel electrophoresis) and DNA digested by a restriction enzyme XhoI was separated by agarose gel electrophoresis and analyzed by Southern blotting.

On a clone cultured in glucose agar media without uracil (no induced expression of SD-Ura, HO endonuclease) and a clone cultured in galactose agar media without leucine (induced expression of SG-Leu, HO endonuclease), their chromosomal DNA separated by PFGE and XhoI digested DNA separated by agarose gel electrophoresis were analyzed using leu2d as a probe. Results are shown in Fig. 5. (A) shows the result for chromosomal DNA separated by PFGE and (B) shows that for XhoI digested DNA separated by agarose gel electrophoresis.

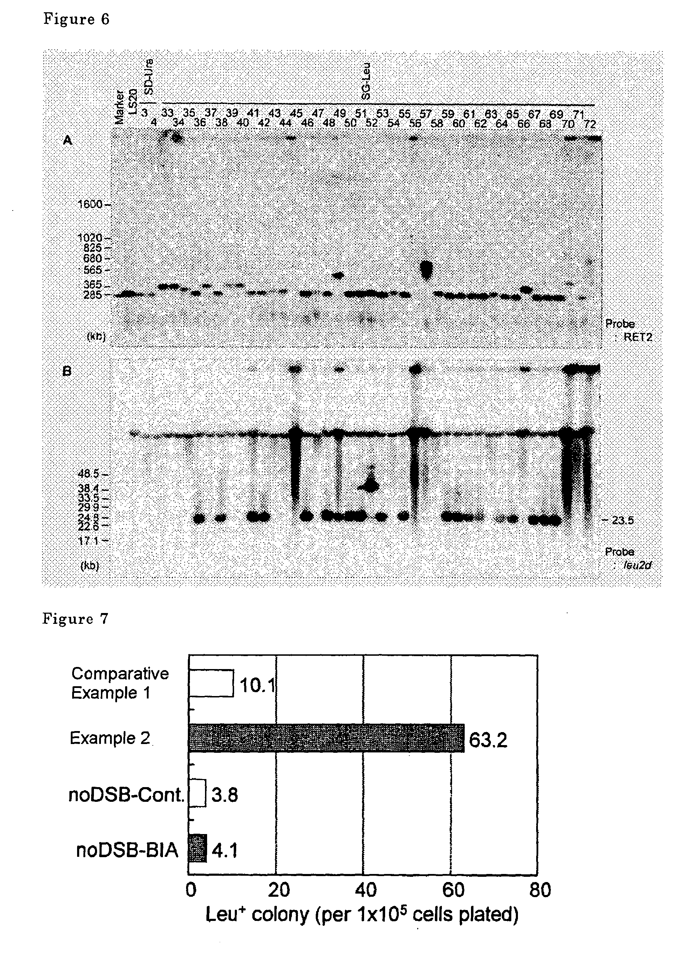

The structure analysis of the amplified product by Southern blotting is shown in Fig. 6. (A) shows the result analyzed by RET2 probe of chromosomal DNA separated by PFGE and (B) shows the result analyzed by leu2d probe of chromosomal DNA separated by FIGE.

According to the analyzed result (Fig. 5), no. 3 chromosome with 345 kb, XhoI fragment with 8.9 kb and about 12.5 kb were detected by leu2d fragment as a probe in host cell strain LS20, in which no. 3 chromosome is partly modified. On the other hand, no. 6 chromosome with 292 kb, in which an amplification unit is inserted, and XhoI fragment with 11.6 kb ascribed to the insertion were detected in negative controls (3 and 4 lane of Fig. 5), in which the expression of HO endonuclease was not induced. In Fig. 5B, XhoI fragments (4.4 kb, 6.4 kb, 8.2 kb, 10.3 kb) are observed as detected by leu2d probe.

In contrast, bands with different sizes from the above results show strong signals in cell strains derived from clones with induced expression of HO endonuclease. Based on the pattern of the signal, these cell strains are classified roughly in 4 groups.
The first group (lane: 35, 43, 44, 47, 54, 58, 63), which shows the pattern expected from the hypothesis of the amplification process, may contain 5 to 7 leu2d in no. 6 chromosome as deduced from the size.

The second group (lane: 45, 49, 56, 57, 66, 70, 72) shows, in PFGE analysis, strong signals in DNA bands with large molecular size, which are unexpected XhoI fragments. Later analysis suggests that these bands contain highly reiterated sequence comprising partly inverted sequence to expected one and they contain more than several tens copies of leu2d.

Also, the analysis with RET2 probe located at the centromere side of the insertion site of amplification unit suggests that those bands are amplified on a chromosome (Fig. 6A). These bands show strong signals in the region of more than about 650kb, i.e. a separation threshold, and near wells in (A) and may reflect extremely high amplification. From the size of the amplified products in (A), it is suggested that clone 66 contains 13 copies, clone 49 contains 29 copies, clone 57 contains 38~54 copies and other clones contain more copies of leu2d. Since those bands in (A) are clear bands, their structure may be relatively stable.

The third group (lane: 36, 38, 42, 46, 48, 50, 51, 53, 55, 59-62, 64, 65, 67-69) shows no change in the size of no. 6 chromosome containing the amplification unit but shows a difference in the pattern of XhoI fragments. FIGE analysis results in the detection of a molecule with about 23.5 kb in this group (Fig. 6B). Later analysis suggests that the molecule is a mini chromosome, in which the sequence of the telomere side of the insertion site of the amplification unit has a palindrome-like structure. The fourth group containing cell strains except the above described may be generated by a rearrangement without leu2d amplification.

As noted in the gene amplification system on a genome of the present invention, particularly high amplification reaction was observed in the second group.

### Comparative Example 1:

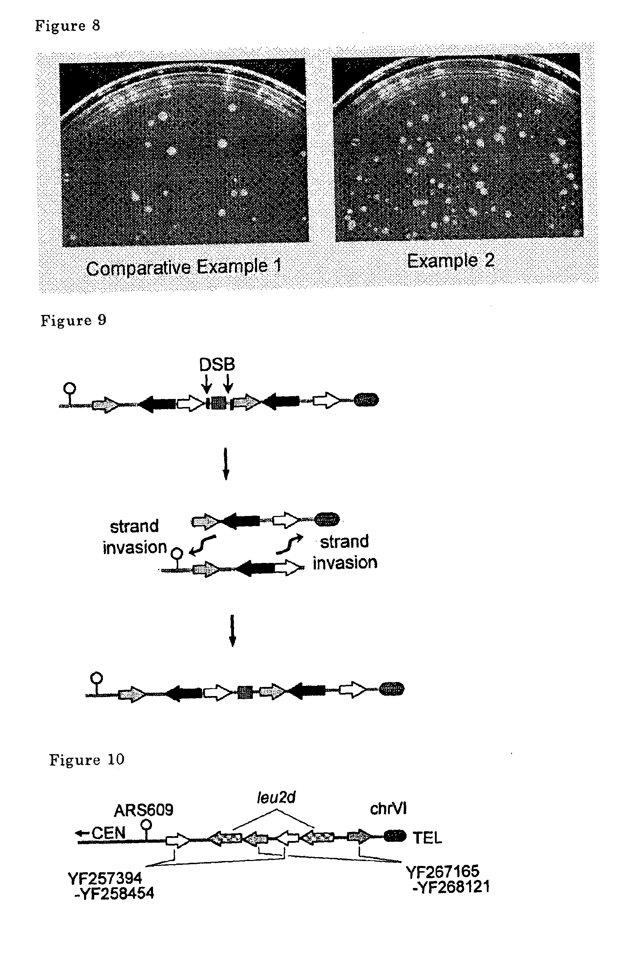

An amplification unit, wherein two kinds of homologous sequences of budding yeast genomes were arranged in the forward orientation each other, was constructed as described in Example 1. The amplification unit was processed in the same way as described in Example 2.
The colony and their cell numbers generated in this Comparative Example 1 and in Example 2 are shown in Figures 7 and 8. According to the colony counting, Example 2 generated about 6.3 times more colonies compared to Comparative Example 1.
In this Comparative Example 1, no BIR reaction might be took place and therefore, no gene replication might be took place.

### Example 3:

An amplification unit was constructed, which comprises two kinds of homologous sequence of budding yeast genomes (YF257394-YF258454 (SEQ ID NO: 3) and YF267165-YF268121 (SEQ ID NO: 4, GeneBank Accession ID; NC 001138) and a selection maker gene leu2d (SEQ ID NO: 5) for amplification as shown the organization in Fig. 10, after URA3 gene (SEQ ID NO: 2) located between two HO sequences (SEQ ID NO: 1) is removed from the amplification unit constructed in Fig. 1.

The amplification unit was processed according to the same way as described in Example 2. Namely, the amplification unit was transduced into budding yeast cell line (LS20) and spread on a glucose medium (without uracil) and on a galactose medium (without leucine). Two colonies (lanes: 7 and 8) and 4 colonies (lanes: 77 to 80) were picked up from the former and the latter plate, respectively, they were grown in the same medium and were analyzed by pulse gel electrophoresis for the examination of chromosomes of each clone. Total culture time was about 4 days.

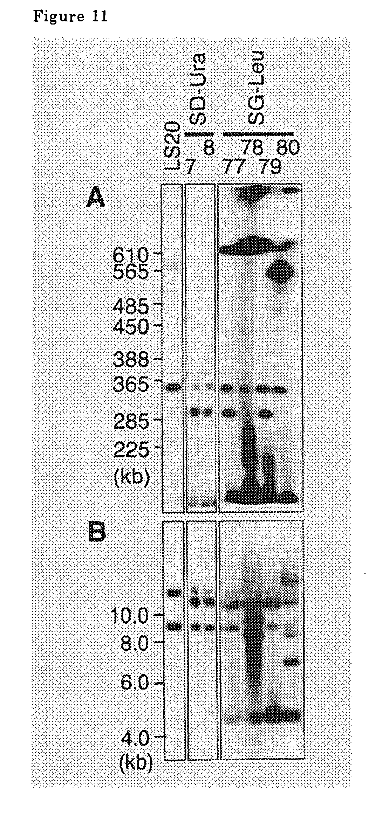

Figure 11 shows the results. In the figure, (A) represents the result of direct electrophoresis, and (B) represents the result of electrophoresis after digestion by restriction enzyme XhoI. The colonies from the former plate show the same pattern to that of the original cells and all the latter colonies show amplification of leu2d. Particularly, clones of lane 78 and 80 show highly amplified leu2d on a chromosome.

### Example 4:

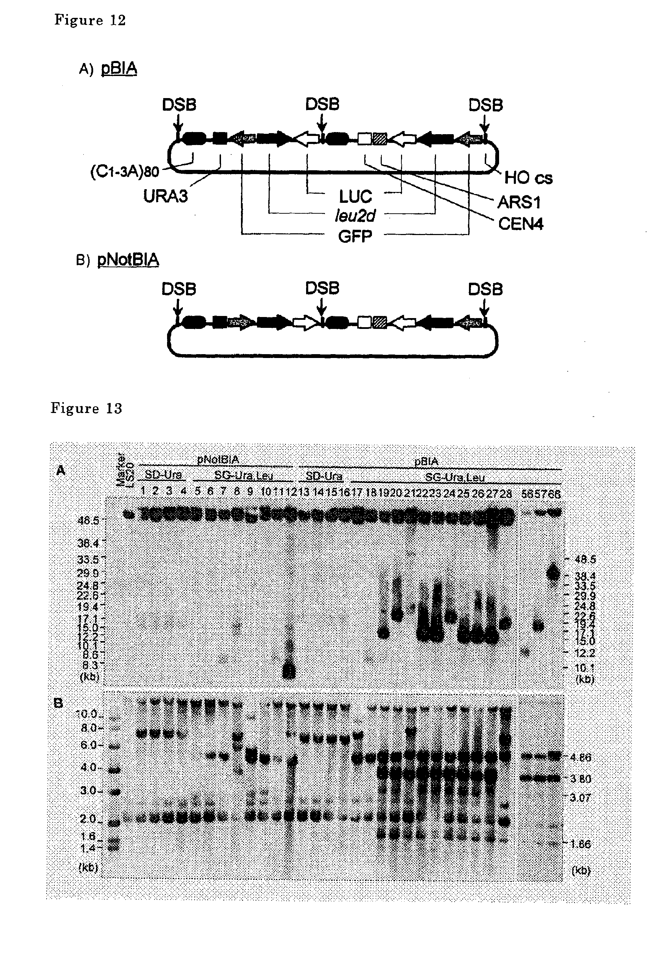

As shown in Fig. 12(A), a plasmid pBIA for amplification was constructed by integrating an amplification unit, which comprises 3 HO sequences (DSB, SEQ ID NO: 1), URA3 gene (SEQ ID NO: 2), ARS1 gene (SEQ ID NO: 6), LUC gene (SEQ ID NO: 7), CEN4 gene (SEQ ID NO: 8), GFP gene (SEQ ID NO: 9) and a selection marker gene leu2d (SEQ ID NO: 5) for amplification..

The plasmid pBIA was transduced into budding yeast cell line (LS20) with a chromosome containing a structure that HO endonuclase gene is inserted into a downstream of GAL promoter, by the use of a transformation kit (Frozen-EZ Yeast Transformation II:ZYMO RESEARCH, Cat No. T2001) based on a lithium acetate method. The yeast cells were grown in media without uracil to a middle log phase, harvested by centrifugation, washed two times with sterilized water, diluted to various concentrations, spread on an agar plate with the same media as above except containing galactose as a sugar source without uracil and leucine, and cultured at 30°C or 25°C. Total culture time was about 4 days. After 4-5 days, colony number was counted. Viable cell number was counted after spread on agar media with complete synthesized glucose (2%).

After the chromosomal DNA was separated by PFGE (pulsed-field gel electrophoresis) or FIGE (field-inversion gel electrophoresis), and DNA digested by restriction enzyme XhoI was separated by agarose gel electrophoresis, they were analyzed by Southern blotting. The result of the structural analysis of the amplified product by Southern blotting is shown in Fig. 13. (A) shows the result of the analysis of chromosomal DNA separated by PFGE with the use of RET2 probe, and (B) shows that of chromosomal DNA separated by FIGE with the use of leu2d probe. SD-Ura represents the clone cultured in glucose media without Ura and SG-Ura, Leu represents the clone selected in Leu-Ura-galactose media.

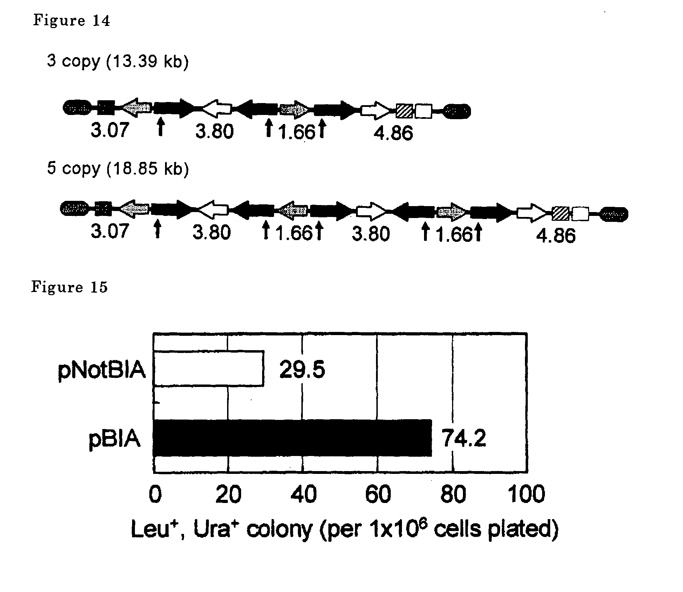

The results of electrophoresis show that leu2d gene was amplified to 3-5 copies by breaking the double strand break site (DSB) of pBIA (a plasmid of the present invention). Circular plasmids pNotBIA and pBIA were observed in a range of size about 17~19 kb of linear DNA in the sample of SD-Ura in Fig. 13(A). In contrast, a unique band is observed only for pBIA sample yet for colonies spread on media without leucine in a range of size about 12~20 kb in the sample of SG-Ura, Leu of Fig. 13(A). Since a linear DNA results generally in more intense band than a circular DNA, the band is ascribed to a linear DNA according to the comparison to the above circular plasmid pBIA. The size of the band is directly related to the amplification and the band with 13.39 kb may imply 3 copies of leu2d gene and that with 18.85 kb may imply 5 copies as shown in Fig. 14.

Figure 13(B) shows the result of agarose gel electrophoresis after the cut by ClaI and the relation between the intensity and the size of the bands is consistent with the above result.

### Comparative Example 2:

A plasmid pNotBIA was constructed as shown the structure in Fig. 12 (B) by the use of a plasmid and genes similar to Example 4. The plasmid was processed in the same way as Example 4. The numbers of Leu⁺Ura⁺ colonies obtained from cell strains (LS20) containing plasmids (pBIA and pNotBIA) of Example 4 and Comparative Example 2 under the induction of galactose were compared. The results are shown in Fig. 15. The figure shows that the use of plasmid pBIA leads to more amplification.

Using the method of the present invention, a high level of amplification was confirmed by examining chromosome and plasmid as a place of amplification (Examples 2 to 4). A clone amplified at least to several tens copies was obtained by the method of the present invention. Among them, some clones were amplified to more than a hundred copies. Integration into a plasmid enables easily to change the structure of the amplification unit and to change the host to be transduced.

## Claims

1. A double-stranded DNA comprising (a) an arrangement of A-C and (b-1) an arrangement of A'-C' or (b-2) an inverted arrangement of A'-C', wherein A and A' are each double-stranded DNA and are capable of undergoing reciprocal homologous recombination and one of A and A' is an inverted orientation of the other, C and C' are each double-stranded DNA and are capable of undergoing reciprocal homologous recombination and one of C and C' is an inverted orientation of the other, and at least one of A and C comprises a target gene for amplification, and any DNA sequence may be inserted among A, A', C and C'.

2. A double-stranded DNA comprising (a) an arrangement of A-B-C and (b-1) an arrangement of A'-B'-C' or (b-2) an inverted arrangement of A'-B'-C', wherein A and A' are each double-stranded DNA and are capable of undergoing reciprocal homologous recombination and one of A and A' is an inverted orientation of the other, B and B' are amplifying segments where at least one of B and B' containing at least one target gene for amplification, C and C' are each double-stranded DNA and are capable of undergoing reciprocal homologous recombination and one of C and C' is an inverted orientation of the other, and any DNA sequence may be inserted among A, A', B, B', C and C'.

3. The double-stranded DNA of claim 2, wherein B and B' are amplifying segments each containing at least one target gene for amplification arranged in the same orientation and are capable of undergoing reciprocal homologous recombination.

4. The double-stranded DNA of claim 3, wherein each of B and B' contains a selection gene for amplification arranged in the same orientation.

5. The double-stranded DNA of claim 1 comprising an arrangement of A-C-A'-C', wherein the symbols are the same as above.

6. The double-stranded DNA of claim 5 comprising an arrangement of A-C-D-A'-C', wherein D represents a double-stranded DNA fragment containing at least one break site by endonuclease and other symbols are the same as above.

7. The double-stranded DNA of any one of claims 2 to 4 comprising an arrangement of A-B-C-A'-B'-C', wherein the symbols are the same as above.

8. The double-stranded DNA of claim 7 comprising an arrangement of A-B-C-D-A'-B'-C', wherein D represents a double-stranded DNA fragment containing at least one break site by endonuclease and other symbols are the same as above.

9. The double stranded DNA of claim 1 comprising (a) an arrangement of E'-A-C and (b-1) an arrangement of A'-C'-E or (b-2) an inverted arrangement of A'-C'-E or (c) an arrangement of A -C-E and (d-1) an arrangement of E'-A'-C' or (d-2) an inverted arrangement of E'-A'-C', wherein E represents a telomere sequence and E' represents an inverted sequence of E and the other symbols are the same as above.

10. The double-stranded DNA of claim 9 comprising an arrangement of D-E'-A-C-D-A'-C'-E-D, D-E'-A-C-D-E'-C"-A"-D, D-A-C-E-D-E'-A'-C'-D or D-A-B-C-E-D-C"-B"-A"-E-D, wherein C"-A" represents an inverted arrangement of A'-C'.

11. The double-stranded DNA of any one of claims 2 to 4 comprising (a) an arrangement of E'-A-B-C and (b-1) an arrangement of A'-B'-C'-E or (b-2) an inverted arrangement of A'-B'-C'-E' or (c) an arrangement of A-B-C-E, and (d-1) an arrangement of E'-A'-B'-C' or (d-2) an inverted arrangement of E'-A'-B'-C', wherein E represents a telomere sequence and E' represents an inverted orientation of E and the other symbols are the same as above.

12. The double-stranded DNA of claim 11 comprising the arrangement of D-E'-A-B-C-D-A'-B'-C'-E-D , D-E'-A-B-C-D-E'-C"-B"-A"-D , D-A-B-C-E-D-E'-A'-B'-C'-D, or D-A-B-C-E-D-C"-B"-A"-E-D, wherein C"-B"-A" represents an inverted arrangement of A'-B'-C'.

13. A recombinant vector containing the double-stranded DNA of any one of claims 1 to 12.

14. A transformant transduced with the double-stranded DNA of any one of claims 1 to 8.

15. A recombinant plasmid integrated with the double-stranded DNA of any one of claims 9 to 12.

16. A method for gene amplification comprising the steps of preparing the transformant of claim 14 and amplifying the target gene.

17. The method for gene amplification of claim 16, wherein the transformant is treated with an endonuclease in the step of amplifying the target gene, when the double-stranded DNA is represented as A-C-D-A'-C' or A-B-C-D-A'-B'-C', wherein the symbols are the same as above.

18. The method for gene amplification comprising the steps of transducing bacteria with the plasmid of claim 15 and culturing the bacteria.

19. The method for producing a protein encoded by the target gene for amplification comprising the steps of culturing cells or bacteria obtained by the method of any one of claims 16 to 18.


SEQUENCE LISTING

<110>  Japan Science and Technology Agency

<120>  A method for gene amplification

<130>  FS04-420PCT

<160>  9

<170>  PatentIn version 3.1

<210>  1
<211>  52
<212>  DNA
<213>  Saccharomyces cerevisiae

<400>  1
gcagcacgga atatgggact acttcgcgca acagtataat tttataaacc ct          52

<210>  2
<211>  1106
<212>  DNA
<213>  Saccharomyces cerevisiae

<400>  2
tttcaattca attcatcatt ttttttttat tctttttttt gatttcggtt tctttgaaat     60

ttttttgatt cggtaatctc cgaacagaag gaagaacgaa ggaaggagca cagacttaga    120

ttggtatata tacgcatatg tagtgttgaa gaaacatgaa attgcccagt attcttaacc    180

caactgcaca gaacaaaaac ctgcaggaaa cgaagataaa tcatgtcgaa agctacatat    240

aaggaacgtg ctgctactca tcctagtcct gttgctgcca agctatttaa tatcatgcac 300

gaaaagcaaa caaacttgtg tgcttcattg gatgttcgta ccaccaagga attactggag 360

ttagttgaag cattaggtcc caaaatttgt ttactaaaaa cacatgtgga tatcttgact 420

gatttttcca tggagggcac agttaagccg ctaaaggcat tatccgccaa gtacaatttt 480

ttactcttcg aagacagaaa atttgctgac attggtaata cagtcaaatt gcagtactct 540

gcgggtgtat acagaatagc agaatgggca gacattacga atgcacacgg tgtggtgggc 600

ccaggtattg ttagcggttt gaagcaggcg gcagaagaag taacaaagga acctagaggc 660

cttttgatgt tagcagaatt gtcatgcaag ggctccctat ctactggaga atatactaag 720

ggtactgttg acattgcgaa gagcgacaaa gattttgtta tcggctttat tgctcaaaga 780

gacatgggtg gaagagatga aggttacgat tggttgatta tgacacccgg tgtgggttta 840

gatgacaagg gagacgcatt gggtcaacag tatagaaccg tggatgatgt ggtctctaca 900

ggatctgaca ttattattgt tggaagagga ctatttgcaa agggaaggga tgctaaggta 960

gagggtgaac gttacagaaa agcaggctgg gaagcatatt tgagaagatg cggccagcaa 1020

aactaaaaaa ctgtattata agtaaatgca tgtatactaa actcacaaat tagagcttca 1080

atttaattat atcagttatt acccgg 1106

<210> 3
<211> 1061

<212> DNA
<213> Saccharomyces cerevisiae

<400> 3
taagtactca agaatgggcc agaaataatg ttagagttta taggggtgac agcccttgag 60

ctaataaata ctacaataac agtgtccgtt tgaatgctgg tcggtgaatc aatcaaagat 120

ttggttgcaa tgatatgtgc aattttgctg atggagcttg ctaaagttgt gatattagtc 180

gagatccaat tagaacggat taggcgggaa tcgcttgagg gatcgccaaa aatacttgtg 240

atttgtgtaa gttggttgta atgcaaacct gctatcttta ctttcgaaca aaatagatga 300

atcagcggct aagtattctg tggctaatgt gctagataca tgttgagtac tgctatttgt 360

gcgcaaccgt tctaacaatg ctgagagatg atgaattgaa tatatcagaa gttttactag 420

ctacagtagt tgaggtacca atagtggggg tatgcgaaaa tttactaaaa gcactgactg 480

gagttgtggt actagtgaat acctggtcca aataggttat gaaggaatca tttgagaatt 540

ggctagaagc aactaaaacg cccgtggatt gaggttcaga tttgctactg tcgctttcga 600

agaagctaga tgaaccacgg gtaaagtatt ctgcatctaa tgtgttcaat aaatattgag 660

tgacgttatc gtaatgttac agtactaaca ccgctagaaa atgctggtgt gaatgtgaat 720

gacgatagac ggactgatgc acttttccat tgtacgataa cattacttac aagattggga 780

gaagcatgat tgaaaatttg actggaagaa ccacttatat taggagtggc ggtattagta 840

gaaaattgac taaacgcatc cgaaaattaa atagaattta aagtttcctt gggtgcactg 900

ttttgggctg cagtgctaaa atccagaagt gttggagtca ggttacttgt ttgcatacag 960

acattactga agttttcaga aggcctttga atcgagaacg agataaggaa gtgtcctcta 1020

aatgcaattt tagagctcaa agtgaggata gtggcactga a 1061

<210> 4
<211> 957
<212> DNA
<213> Saccharomyces cerevisiae

<400> 4
ctagcatcaa agacgggtgt tggaatttga caatttgctt caatgtatcc aaggaagttt 60

taggtaatgg caggctaaat cttccaatca aacagcaaaa tacacctcaa gacgccccaa 120

gaattttgaa tgaaggcgag ctgaatcccg aactttgttc tttaatgtac aatttaatac 180

caaaatgatt atccaacttg attagcttcg atgactcata cagatccata ccaatatcaa 240

cttcacggat aactttttca actcttttaa attcttcaac atcatttttc gatcaaactg 300

cgtcaggttt tacatataga tcaagcgttg tcggtttata ctacgatgtc atttacgatc 360

tgctgccaac agaagaaaca ttacgcagta ccttggaaaa atctaggcaa tttatagaca 420

tgaatctcct atcgttctta aaacaatgta tttcctgtcg ttgtctcgat tctctttttt 480

gctattctga aggacaggtc catttatcat ccagaattct aatcccgaat ttaagatatc 540

gtaacagtaa caacatcata tccaagcacc gaagattcat tgctctatat tccttgtctt 600

cgaataagga agataccgcg aagagactct ttaggagtcg ggcttgactc cctgccaaag 660

cagctattcc tgtataacac gcgatatttt atctccttgt cgtcatgtga aagtcaaaca 720

gcgcacgttt gtttgatgaa gcagcaactc tcctcttact gcttttgcag aattttgtga 780

gaagctctct tctcttggaa ctaacctagc catatggctt tctgccctct ttccgtcaac 840

attacaagat aaagatatcg gtataattta aagattcgac ctttacgccc tacatggcta 900

aaattacaat gccaaaatta tcatgaattc actttgttga cccggaatta caataca 957

<210> 5
<211> 1953
<212> DNA
<213> Saccharomyces cerevisiae

<400> 5
gatctagagg ttaactaagc gaatttctta tgatttatga tttttattat taaataagtt 60

ataaaaaaaa taagtgtata caaattttaa agtgactctt aggttttaaa acgaaaattc 120

ttattcttga gtaactcttt cctgtaggtc aggttgcttt ctcaggtata gcatgaggtc 180

gctcttattg accacatctc taccggcatg ccgagcaaat gcctgcaaat cgctccccat 240

ttcacccaat tgtagatatg ctaactccag caatgagttg atgaatctcg gtgtgtattt 300

tatgtcctca gaggacaaca cctgttgtaa tcgttcttcc acacggatct tatatatatt 360

tcaaggatat accattctaa tgtctgcccc taagaagatc gtcgttttgc caggtgacca 420

cgttggtcaa gaaatcacag ccgaagccat taaggttctt aaagctattt ctgatgttcg 480

ttccaatgtc aagttcgatt tcgaaaatca tttaattggt ggtgctgcta tcgatgctac 540

aggtgtccca cttccagatg aggcgctgga agcctccaag aaggttgatg ccgttttgtt 600

aggtgctgtg ggtggtccta aatggggtac cggtagtgtt agacctgaac aaggtttact 660

aaaaatccgt aaagaacttc aattgtacgc caacttaaga ccatgtaact ttgcatccga 720

ctctctttta gacttatctc caatcaagcc acaatttgct aaaggtactg acttcgttgt 780

tgtcagagaa ttagtgggag gtatttactt tggtaagaga aaggaagacg atggtgatgg 840

tgtcgcttgg gatagtgaac aatacaccgt tccagaagtg caaagaatca caagaatggc 900

cgctttcatg gccctacaac atgagccacc attgcctatt tggtccttgg ataaagctaa 960

tgttttggcc tcttcaagat tatggagaaa aactgtggag gaaaccatca agaacgaatt 1020

ccctacattg aaggttcaac atcaattgat tgattctgcc gccatgatcc tagttaagaa 1080

cccaacccac ctaaatggta ttataatcac cagcaacatg tttggtgata tcatctccga 1140

tgaagcctcc gttatcccag gttccttggg tttgttgcca tctgcgtcct tggcctcttt 1200

gccagacaag aacaccgcat ttggtttgta cgaaccatgc cacggttctg ctccagattt 1260

gccaaagaat aaggtcaacc ctatcgccac tatcttgtct gctgcaatga tgttgaaatt 1320

gtcattgaac ttgcctgaag aaggtaaggc cattgaagat gcagttaaaa aggttttgga 1380

tgcaggtatc agaactggtg atttaggtgg ttccaacagt accacggaag tcggtgatgc 1440

tgtcgccgaa gaagttaaga aaatccttgc ttaaaaagat tctctttttt tatgatattt 1500

gtacataaac tttataaatg aaattcataa tagaaacgac acgaaattac aaaatggaat 1560

atgttcatag ggtagacgaa actatatacg caatctacat acatttatca agaaggagaa 1620

aaaggaggat gtaaaggaat acaggtaagc aaattgatac taatggctca acgtgataag 1680

gaaaaagaat tgcactttaa cattaatatt gacaaggagg agggcaccac acaaaaagtt 1740

aggtgtaaca gaaaatcatg aaactatgat tcctaattta tatattggag gattttctct 1800

aaaaaaaaaa aaatacaaca aataaaaaac actcaatgac ctgaccattt gatggagttt 1860

aagtcaatac cttcttgaac catttcccat aatggtgaaa gttccctcaa gaattttact 1920

ctgtcagaaa cggccttaac gacgtagtcg acg 1953

<210> 6
<211> 742
<212> DNA
<213> Saccharomyces cerevisiae

<400> 6
ttgatgaggc aacgctaatt atcaacatat agattgttat ctatctgcat gaacacgaaa 60

tctttacttg acgacttgag gctgatggtg tttatgcaaa gaaaccactg tgtttaatat 120

gtgtcactgt ttgatattac tgtcagcgta gaagataata gtaaaagcgg ttaataagtg 180

tatttgagat aagtgtgata aagtttttac agcgaaaaga cgataaatac aagaaaatga 240

ttacgaggat acggagagag gtatgtacat gtgtatttat atactaagct gccggcggtt 300

gtttgcaaga ccgagaaaag gctagcaaga atcgggtcat tgtagcgtat gcgcctgtga 360

acattctctt caacaagttt gattccattg cggtgaaatg gtaaaagtca accccctgcg 420

atgtatattt tcctgtacaa tcaatcaaaa agccaaatga tttagcatta tctttacatc 480

ttgttatttt acagatttta tgtttagatc ttttatgctt gcttttcaaa aggcctgcag 540

gcaagtgcac aaacaatact taaataaata ctactcagta ataacctatt tcttagcatt 600

tttgacgaaa tttgctattt tgttagagtc ttttacacca tttgtctcca cacctccgct 660

tacatcaaca ccaataacgc catttaatct aagcgcatca ccaacatttt ctggcgtcag 720

tccaccagct aacataaaat gt 742

<210> 7
<211> 937
<212> DNA
<213> pGL3-Promoter

<400> 7
ccagagtcct tcgataggga caagacaatt gcactgatca tgaactcctc tggatctact 60

ggtctgccta aaggtgtcgc tctgcctcat agaactgcct gcgtgagatt ctcgcatgcc 120

agagatccta tttttggcaa tcaaatcatt ccggatactg cgattttaag tgttgttcca 180

ttccatcacg gttttggaat gtttactaca ctcggatatt tgatatgtgg atttcgagtc 240

gtcttaatgt atagatttga agaagagctg tttctgagga gccttcagga ttacaagatt 300

caaagtgcgc tgctggtgcc aaccctattc tccttcttcg ccaaaagcac tctgattgac 360

aaatacgatt tatctaattt acacgaaatt gcttctggtg gcgctcccct ctctaaggaa 420

gtcggggaag cggttgccaa gaggttccat ctgccaggta tcaggcaagg atatgggctc 480

actgagacta catcagctat tctgattaca cccgaggggg atgataaacc gggcgcggtc 540

ggtaaagttg ttccattttt tgaagcgaag gttgtggatc tggataccgg gaaaacgctg 600

ggcgttaatc aaagaggcga actgtgtgtg agaggtccta tgattatgtc cggttatgta 660

aacaatccgg aagcgaccaa cgccttgatt gacaaggatg gatggctaca ttctggagac 720

atagcttact gggacgaaga cgaacacttc ttcatcgttg accgcctgaa gtctctgatt 780

aagtacaaag gctatcaggt ggctcccgct gaattggaat ccatcttgct ccaacacccc 840

aacatcttcg acgcaggtgt cgcaggtctt cccgacgatg acgccggtga acttcccgcc 900

gccgttgttg ttttggagca cggaaagacg atgacgg 937

<210> 8
<211> 1062
<212> DNA
<213> Saccharomyces cerevisiae

<400> 8
tgggccattc tcatgaagaa tatcttgaat ttattgtcat attactagtt ggtgtggaag 60

tccatatatc ggtgatcaat atagtggttg acatgctggc tagtcaacat tgagcctttt 120

gatcatgcaa atatattacg gtattttaca atcaaatatc aaacttaact attgacttta 180

taacttattt aggtggtaac attcttataa aaaagaaaaa aattactgca aaacagtact 240

agcttttaac ttgtatccta ggttatctat gctgtctcac catagagaat attacctatt 300

tcagaatgta tgtccatgat tcgccgggta aatacatata atacacaaat ctggcttaat 360

aaagtctata atatatctca taaagaagtg ctaaattggc tagtgctata tatttttaag 420

aaaatttctt ttgactaagt ccatatcgac tttgtaaaag ttcactttag catacatata 480

ttacacgagc cagaaattgt aacttttgcc taaaatcaca aattgcaaaa tttaattgct 540

tgcaaaaggt cacatgctta taatcaactt ttttaaaaat ttaaaatact tttttatttt 600

ttatttttaa acataaatga aataatttat ttattgttta tgattaccga aacataaaac 660

ctgctcaaga aaaagaaact gttttgtcct tggaaaaaaa gcactaccta ggagcggcca 720

aaatgccgag gctttcatag cttaaactct ttacagaaaa taggcattat agatcagttc 780

gagttttctt attcttcctt ccggttttat cgtcacagtt ttacagtaaa taagtatcac 840

ctcttagagt taactatgag ataagcaagt atcatctcat ttcatttacc tgaagtcgag 900

taaacagaaa atccaattgt tgatgaacct caatgactta gaactatcta tcggcagatc 960

atataaagag gatttaggta cctagaggac tgtacctgga gtatatatat atatatatat 1020

atattatctc aactatagtc catagaggtt tctttcttga gg 1062

<210> 9
<211> 600
<212> DNA
<213> pEGFP-N1

<400> 9
gcgacgtaaa cggccacaag ttcagcgtgt ccggcgaggg cgagggcgat gccacctacg 60
gcaagctgac cctgaagttc atctgcacca ccggcaagct gcccgtgccc tggcccaccc 120
tcgtgaccac cctgacctac ggcgtgcagt gcttcagccg ctaccccgac cacatgaagc 180
agcacgactt cttcaagtcc gccatgcccg aaggctacgt ccaggagcgc accatcttct 240
tcaaggacga cggcaactac aagacccgcg ccgaggtgaa gttcgagggc gacaccctgg 300
tgaaccgcat cgagctgaag ggcatcgact tcaaggagga cggcaacatc ctggggcaca 360
agctggagta caactacaac agccacaacg tctatatcat ggccgacaag cagaagaacg 420
gcatcaaggt gaacttcaag atccgccaca acatcgagga cggcagcgtg cagctcgccg 480
accactacca gcagaacacc cccatcggcg acggccccgt gctgctgccc gacaaccact 540
acctgagcac ccagtccgcc ctgagcaaag accccaacga gaagcgcgat cacatggtcc 600